# EUROPEAN PATENT APPLICATION

(11) **EP 4 159 881 A1**
(43) Date of publication of application: **05.04.2023**
(21) Application number: 21813087.0
(22) Date of filing: 19.05.2021
(51) Int. Cl.: C22B 3/32, C22B 7/00, C22B 23/00, C22B 47/00, C22B 59/00

(54) **APPLICATIONS OF CARBOXYLIC COMPOUND SERVING AS EXTRACTING AGENT AND METAL ION EXTRACTION METHOD**

(30) Priority: 27.05.2020 CN 202010463421
(71) Applicant: Botree Cycling Sci & Tech Co., Ltd., Beijing 100083 (CN)
(72) Inventor: WANG, Xue, Beijing 100083 (CN)
(74) Representative: Cesa, Roberta
(86) International application number: PCT/CN2021/094590
(87) International publication number: WO 2021/238738

(57) **Abstract**

Disclosed are applications of a carboxylic compound serving as an extracting agent and a metal ion extraction method. The carboxylic compound is provided with the structure as represented by formula I. The extracting agent as represented by formula I is characterized by a secondary atom at position α of the carboxyl group, in distinction from a primary carbon carboxylic acid at position α and a tertiary carbon carboxylic acid at position α, the presence of a secondary carbon carboxylic acid provides a proper steric hindrance, provides improved selectivity with respect to ions, and provides a high separation coefficient, low stripping acidity, and high load rate when used for the extraction and separation of metal ions; moreover, the carboxylic compound of formula I has great stability and low aqueous solubility, allows an extraction process to be stable, reduces environmental pollution, reduces costs, and provides significant application prospects.

## Description

### TECHNICAL FIELD

The present application belongs to the technical field of hydrometallurgy and relates to an application of a carboxylic acid compound as an extractant and a metal ion extraction method, and for example, relates to an application of a carboxylic acid compound as an extractant in hydrometallurgy and a metal ion extraction method.

### BACKGROUND

With the advantages of good selectivity, high metal recovery and fast mass transfer, the solvent extraction method is an important part of industrial enrichment, refining, separation and purification of valuable metals such as non-ferrous metals and rare earth elements, which has been continuously focused on and developed by many researchers. However, the urgency of environmental protection and resource recycling has demanded higher requirements for the performance of extraction systems in terms of energy consumption, acid consumption, effluent discharge and production capacity. There has been a strong and real demand for extractants with better performance in order to meet the higher requirements.

Although a large number of extractants such as phosphorus, amines and carboxylic acids are available in the field of solvent extraction at present, the existing extractants no longer satisfies the requirements from new raw materials, components to be separated, environment or economic costs as society develops. Therefore the research related to improving existing extraction systems or developing new extraction systems has received much attention. For example, although P507/P204 hydrochloric acid system is widely used for the separation of rare earths, the P507/P204 hydrochloric acid system has poor regeneration performance for heavy rare earths, high back extraction acidity and serious pollution; C272 is used for the separation of nickel and cobalt, but for the recovery of nickel-cobalt-manganese ternary positive electrode material of novel lithium-ion batteries, C272 requires a complex process due to the priority extraction of calcium and magnesium before nickel; the performance of "naphthenic acid" can no longer meet the requirements of extraction and purification of yttrium from rare earth mixtures, and extractants that can replace naphthenic acid need to be developed; amine extractants such as N1923 and N235 will extract acid during the metal extraction, easily leading to three phases, and the process is difficult to control.

Compared with phosphorus and amine extractants, the carboxylic acids have obvious features of low cost, abundant sources, low acid consumption, and more environmental friendliness when used in metal ion extraction. A variety of carboxylic acid extractants have been reported for metal extraction in recent years, such as tertiary carbon carboxylic acid Versatic 10 and Versatic 911 (CN110029226A Method for recovering valuable metal from used ternary lithium-ion positive electrode material), neodecanoic acid and alkoxy acetic acid (CN93112500.6 Extractant for separating rare-earth metal). However, Versatic 10, Versatic 911 and neodecanoic acid have relatively large solubility in the aqueous phase whenever used in extracting non-ferrous metals such as nickel, cobalt and manganese or extracting rare earths, resulting in process instability, environmental pollution, high cost and product purification difficulty; alkoxy acetic acid is tentatively selected to replace naphthenic acid for the extraction and separation of yttrium (Y³⁺ ) from rare earth element mixture, and unfortunately, the esterification reaction will occur between the alkoxy acetic acid and alcohol phase modifier during the extraction (Yanliang Wang Deqian Li et al. Separation and Purification Technology 82 (2011) 197-201), and the effective extractant concentration will gradually decrease, and no industrial application has been achieved yet.

The above shows that although the carboxylic acid extractants have many unique advantages compared with phosphorus and amine extractants, the currently reported carboxylic acid extractants have greatly limited in application or development due to their solubility and stability. Therefore, the new high-efficiency carboxylic acid extractants have promising application prospects and great economic, environmental and social value.

### SUMMARY

A first object of the present application is to provide an application of a carboxylic acid compound as an extractant, and in particular to provide an application of a carboxylic acid compound as an extractant in hydrometallurgy. The carboxylic acid compound used as an extractant has good ion selectivity, low back extraction acidity, high stability, low water solubility and low cost.

To achieve this object, the present application adopts the technical solutions below.

The present application provides an application of a carboxylic acid compound as an extractant, and the carboxylic acid compound has a structure shown in formula I:

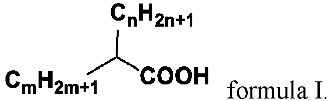

In formula I, 10≤m + n≤22, such as 11, 12, 13, 14, 15, 16, 17, 18, 19, 20 or 21, and both m and n are positive integers.

The carboxylic acid extractant shown in formula I can be extracted from natural substances or synthesized by conventional methods, and the extractant can be a mixture of one or more carboxylic acids when used for extraction.

Exemplarily, the compound of formula I can be prepared with reference to the Jones oxidation reaction, and namely, the corresponding alcohol of the compound of formula I is oxidized by chromic acid to carboxylic acid and ketone in acetone. The oxidizing agent in this reaction is also referred to as Jones reagent, which is a solution of chromium trioxide in concentrated sulfuric acid. A synthetic route of the compound of formula I is as follows:

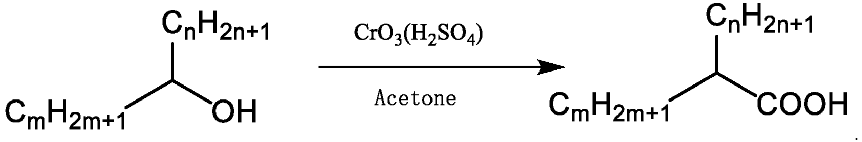

Optionally, 10≤m + n≤ +.

Optionally, the m and the n are each independently an integer selected from 2 to 20, such as 3, 4, 5, 6, 7, 8, 9, 10, 11, 12, 13, 14, 15, 16, 17, 18 or 19.

Optionally, the m and the n are each independently an integer selected from 2 to 10.

Optionally, the carboxylic acid compound includes any one or a combination of at least two of the following compounds: 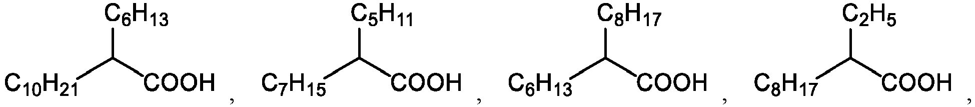 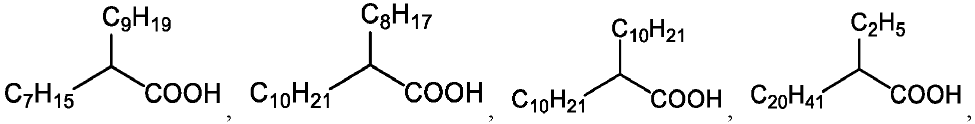 or

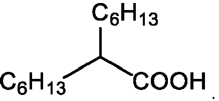

Optionally, the carboxylic acid compound is applied to extraction and separation of a metal ion.

The present application optionally uses the extractant of formula I for the metal ion extraction, due to the fact that the compound of formula I is characterized by a secondary carbon in the α-position to the carboxyl group. The secondary carbon carboxylic acid has proper steric hindrance, which is distinguished from the α-primary carbon carboxylic acid and the α-tertiary carbon carboxylic acid, and has better ion selectivity, thus achieving effective extraction and separation of metal ions.

Optionally, the metal ion includes any one or a combination of at least two of Fe³⁺, Al³⁺, Cu²⁺, Zn²⁺, Cd²⁺, Ni²⁺, Co²⁺, Mn²⁺, Ca²⁺, Mg²⁺, Li⁺, Na⁺, K⁺, Cr³⁺, Ga³⁺, In³⁺, Ti⁴⁺, Sc³⁺, Y³⁺, La³⁺, Ce³⁺, Pr³⁺, Nd³⁺, Sm³⁺, Eu³⁺, Gd³⁺, Tb³⁺, Dy³⁺, Ho³⁺, Er³⁺, Tm³⁺, Yb³⁺ or Lu³⁺.

Without contradicting the common knowledge in the field, the extraction and separation of the above elements will be accompanied by common ions in a feed solution. The above optional conditions can be combined in any way to obtain various preferred examples of the present application.

Optionally, the metal ion includes a non-ferrous metal ion and/or a rare earth metal ion.

Optionally, in the extraction and separation, an anion to match the metal ion in a feed solution to be extracted includes any one or a combination of at least two of Cl⁻, SO₄²⁻ or NO₃⁻.

Optionally, the carboxylic acid compound is applied to the extraction and separation of the metal ion from a used lithium-ion battery positive electrode material, a nickel-cobalt-containing waste residue or nickel laterite ore.

A second object of the present application is to provide an extracting organic phase, and the extracting organic phase includes the carboxylic acid compound shown in formula I:

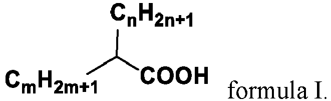

In formula I, 10mu + n≤22, such as 11, 12, 13, 14, 15, 16, 17, 18, 19, 20 or 21, and both m and n are positive integers.

Optionally, the extracting organic phase further includes a diluent.

Optionally, the extracting organic phase further includes a diluent, and the diluent optionally includes any one or a combination of at least two of solvent oil, kerosene, toluene, Escaid 110, hexane, heptane or dodecane; the solvent oil includes solvent oil No. 200 or 260 (i.e., sulfonated kerosene), and the dodecane is n-dodecane.

Optionally, in the extracting organic phase, the carboxylic acid compound shown in formula I has a concentration of 0.1-2.0 mol/L, such as 0.2 mol/L, 0.3 mol/L, 0.4 mol/L, 0.8 mol/L, 1.0 mol/L or 2.0 mol/L.

A third object of the present application is to provide a metal ion extraction method, and the extraction method includes the following steps:
extracting a metal ion containing phase to be extracted by using the extracting organic phase according to the second object, and back-extracting an obtained loaded organic phase to obtain a metal ion enriched solution and a regenerated organic phase.

Optionally, a back extractant for the back-extracting includes hydrochloric acid and/or sulfuric acid.

Optionally, the hydrochloric acid used for the back-extracting has a concentration of 1-4 mol/L, such as 1.5 mol/L, 2 mol/L, 2.5 mol/L, 3 mol/L or 3.5 mol/L.

Optionally, the sulfuric acid used for the back-extracting has a concentration of 0.5-4 mol/L, such as 1 mol/L, 2 mol/L or 3 mol/L.

Optionally, the extracting organic phase and the phase to be extracted has a volume ratio (O/A) of 1:10-10:1, such as 2:9, 3:8, 4:7, 5:6, 6:5, 7:4, 8:3 or 9:2.

Optionally, the loaded organic phase and the back extractant has a volume ratio (O/A) of 1:10-10:1, such as 2:9, 3:8, 4:7, 5:6, 6:5, 7:4, 8:3 or 9:2; the back extraction can be carried out one or more times.

Compared with the prior art, the present application has the following beneficial effects:
(1) When applied to the extraction and separation of metal ions, the extractant shown in formula I has a high separation coefficient (about 20-30% higher compared to Versatic 10), low back extraction acidity (the back extraction rate is greater than 99% by using 1.0 mol/L hydrochloric acid for back extraction), high loading rate (the saturation capacity for Ni²⁺ is about 16.9 g/L), etc;
(2) The carboxylic acid compound shown in formula I has high stability, and low water solubility (the oil content at the extraction equilibrium at pH 8-9 is about 100 mg/L), which guarantees a stable extraction process and can reduce environmental pollution and cost;
(3) The extractant shown in formula I has low cost and promising application prospects, which can be used in various systems such as ternary battery recycling and battery-grade nickel sulfate preparation.

### BRIEF DESCRIPTION OF DRAWINGS

FIG. 1 shows extraction rate E%-pH curves of Extractant 191 for each ion in Example 1.
FIG. 2 shows extraction rate E%-pH curves of Extractant 195 for each ion in Example 2.
FIG. 3 shows extraction rate E%-pH curves of Extractant 196 for each ion at an O/A condition of 5:1 in Example 3.
FIG. 4 shows extraction rate E%-pH curves of Extractant 196 for each ion at an O/A condition of 8:1 in Example 3.
FIG. 5 shows extraction rate E%-pH curves of Extractant 196 for each ion in Example 4.
FIG. 6 shows extraction rate E%-pH curves of Extractant 191 for nickel and magnesium ions in Example 5.
FIG. 7 shows extraction rate E%-pH curves of stearic acid for each ion in Comparative Example 4.

### DETAILED DESCRIPTION

Embodiments are described below in the present application to understand the present application. It should be apparent to those skilled in the art that the embodiments are merely used for a better understanding of the present application and should not be regarded as a specific limitation of the present application.

The information about experiments in the embodiments is as follows:.

Potentiometric titration for acid content of the product, with reference to the literature: Yuan Chengye, Hu Shuisheng; Studies on Organophosphorus Compounds XVI. Substituent Constants σp for Long Chain Alkyl and Alkoxyl Groups and their Correlation with Group Connectivity [J]. Acta Chimica Sinica, 1986, 44, 590-596; potentiometric titrator: Metrohm 907 Titrando, Switzerland.

In the embodiments of the present application, an aqueous phase is prepared by the conventional method: a certain amount of salt is weighed out (the salt is selected according to the acid to be used in back extraction; for example, if sulfuric acid is going to be used in the back extraction, iron sulfate can be selected), dissolved in deionized water and diluted to a certain concentration.

In the embodiments of the present application, the term "saponification" refers to the replacement of a hydrogen ion in the carboxylic acid extractant by an alkali metal ion and/or NH₄⁺ (exchange with a metal ion to achieve extraction); the saponification proportion refers to the proportion of the alkali metal and/or NH₄⁺ to the original hydrogen ion; the steps includes mixing an organic phase and an aqueous solution of base. The aqueous solution of base has a molar concentration of 6 mo1/L to 14 mol/L. The base can be the conventional base in the art, preferably an inorganic base and/or an organic base. The inorganic base is preferably sodium hydroxide and/or potassium hydroxide. The organic base is preferably ammonia.

In the embodiments of the present application, after extraction, a metal ion concentration of the aqueous phase is analyzed using the inductively coupled plasma optical emission spectroscopy (ICP-OES), and a metal ion concentration of the organic phase is calculated by the difference subtraction method.

Raw materials for which no preparation method is provided in the embodiments are commercially available.

Synthesis Example 1 exemplarily gives a specific preparation method and characterization data of a carboxylic acid compound shown in formula I.

### Synthesis Example 1

### Synthesis of Extractant 196

Steps: 153 g of hexadecanol (about 200 mL) was added in a round bottom flask, 300 mL of acetone (1.5 times the volume) was added, and then Jones reagent was slowly added; the solution had a temperature of 8°C at the beginning, and then became green after adding; when the temperature reached 14°C, the drop speed was slowed down, and the temperature was constant at 14°C; when the temperature dropped to 13°C, the drop speed was speeded up, and the solution was stirred constantly; the temperature rising indicated that the reaction was still continuing; meanwhile, the solution that had no phase separation could be observed; after adding ice, the temperature was stable at 15°C; the stirring was speeded up, the temperature would rise continually, the stirring was stopped, and the temperature was stable at 19°C; the product was obtained by spotting a thin layer chromatography plate; the product was dissolved with dichloromethane, and then washed with dilute acid, dilute base and distilled water, respectively; after the oil and aqueous phases separating, dichloromethane was removed by rotary evaporation to obtain Extractant 196.

Characterization data:¹³C NMR (101 MHz, CDCl₃) δ 183.50 (s), 77.43-76.83 (m), 76.67 (s), 45.65 (s), 32.31-31.38 (m), 29.37 (dd, J=25.0, 8.9 Hz), 27.35 (d, J=3.4 Hz), 22.63 (d, J=5.9 Hz), 14.02 (d, J=4.4 Hz); ¹H NMR (400 MHz, CDCl₃) δ 2.24 (1H), 1.70 (4H), 1.45 (20H), 0.85 (6H); MS: 256.2.

### Example 1

### Extraction performance of Extractant 191 for non-ferrous metal ions

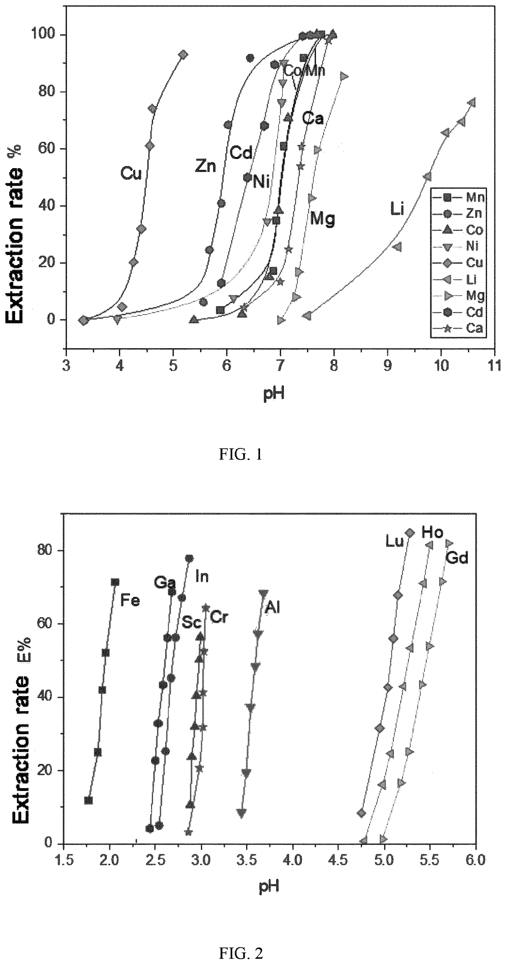

Extractant 191 has a structure: 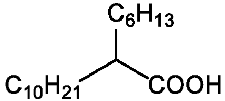 (m=10, n=6, and an acid content is 90%). Extractant 191 was dissolved in dodecane to prepare a 0.2 mol/L organic phase, and 0.01 mol/L sulfate solutions of Cu²⁺, Zn²⁺, Cd²⁺, Ni²⁺, Co²⁺, Mn²⁺, Ca²⁺, Mg²⁺, and Li⁺ were prepared as aqueous phases, respectively. With a condition that the organic phase and the aqueous phase had a ratio of 1:1, and controlling the pH by saponification using 6 mol/L sodium hydroxide (Table 1), the extraction was carried out, and then the extraction rate E%-pH curves of Extractant 191 for each ion were obtained (FIG. 1), and the separation coefficients of Extractant 191 between various ions are shown in Table 2.

**Table 1: Extraction pH_{1/2} of Extractant 191 for each ion**

| | Cu²⁺ | Zn²⁺ | Cd²⁺ | Ni²⁺ | Co²⁺ | Mn²⁺ | Ca²⁺ | Mg²⁺ | Li⁺ |
|---|---|---|---|---|---|---|---|---|---|
| pH_{1/2} | 4.49 | 5.91 | 6.38 | 6.85 | 7.00 | 7.01 | 7.32 | 7.59 | 9.75 |

**Table 2: Separation coefficients of Extractant 191 between various ions**

| | Cu²⁺ | Zn²⁺ | Cd²⁺ | Ni²⁺ | Co²⁺ | Mn²⁺ | Ca²⁺ |
|---|---|---|---|---|---|---|---|
| Zn²⁺ | 691.83 | | | | | | |
| Cd²⁺ | 6025.60 | 8.71 | | | | | |
| Ni²⁺ | 52480.75 | 75.86 | 8.71 | | | | |
| Co²⁺ | 104712.85 | 151.36 | 17.38 | 2.00 | | | |
| Mn²⁺ | 109647.82 | 158.49 | 18.20 | 2.09 | 1.05 | | |
| Ca²⁺ | 457088.19 | 660.69 | 75.86 | 8.71 | 4.37 | 4.17 | |
| Mg²⁺ | 1584893.19 | 2290.87 | 263.03 | 30.20 | 15.14 | 14.45 | 3.47 |

It can be seen from Table 2 that when Extractant 191 was used for extraction and separation, the separation coefficients between various ions, such as Cu²⁺, Zn²⁺, Cd²⁺, Ni²⁺, Co²⁺, Mn²⁺, Ca²⁺, Mg²⁺, and Li⁺, are all greater than 1, and the separation can be achieved.

FIG. 1 shows that Extractant 191 extracts nickel and cobalt ions in order before calcium and magnesium, and the separation coefficients between nickel and magnesium or between cobalt and magnesium are high, so that this extractant can be applied to the separation of non-ferrous metal ions and impurity metal ions.

### Example 2

Extraction performance of Extractant 195 for trivalent ions

Extractant 195 has a structure: 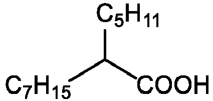 (m=7, n=5, and an acid content is 93%).

The Extractant 195 was dissolved in toluene to prepare a 0.1 mol/L organic phase, and 0.005 mol/L chloride solutions of Fe³⁺, Ga³⁺, In³⁺, Sc³⁺, Cr³⁺, Al³⁺, Lu³⁺, Ho³⁺, and Gd³⁺ were prepared as aqueous phases, respectively. With a condition that the organic phase and the aqueous phase had a ratio of 1:1, and controlling the pH by saponification using 8 mol/L sodium hydroxide (Table 3), the extraction was carried out, and then the extraction rate E%-pH curves of Extractant 195 for each ion were obtained (FIG. 2).

**Table 3: Extraction pH_{1/2} of Extractant 195 for each ion**

| | Fe³⁺ | Ga³⁺ | In³⁺ | Sc³⁺ | Cr³⁺ | Al³⁺ | Lu³⁺ | Ho³⁺ | Gd³⁺ |
|---|---|---|---|---|---|---|---|---|---|
| pH_{1/2} | 1.94 | 2.60 | 2.69 | 2.97 | 3.03 | 3.60 | 5.06 | 5.26 | 5.46 |

**Table 4: Separation coefficients of Extractant 195 between various ions**

| | Fe³⁺ | Ga³⁺ | In³⁺ | Sc³⁺ | Cr³⁺ | Lu³⁺ | Ho³⁺ |
|---|---|---|---|---|---|---|---|
| Ga³⁺ | 95.50 | | | | | | |
| In³⁺ | 177.83 | 1.86 | | | | | |
| Sc³⁺ | 1230.27 | 12.88 | 6.92 | | | | |
| Cr³⁺ | 1862.09 | 19.50 | 10.47 | 1.51 | | | |
| Al³⁺ | 95499.26 | 1000.00 | 537.03 | 77.62 | 51.29 | | |
| Ho³⁺ | / | / | / | / | / | 3.98 | |
| Gd³⁺ | / | / | / | / | / | 15.85 | 3.98 |

It can been seen from FIG. 2 and Table 4 that when Extractant 195 was used for extraction and separation, the separation coefficients between various ions, such as Fe³⁺, Ga³⁺, In³⁺, Sc³⁺, Cr³⁺, Al³⁺, Lu³⁺, Ho³⁺ and Gd³⁺, are all greater than 1, and each rare earth ion can be effectively separated, indicating that this extractant can be applied to the separation of rare earth ions.

### Example 3

### Extraction performance of Extractant 196 for Ni/Co/Mn/Ca/Mg mixed ions in a battery material solution

Extractant 196 has a structure: 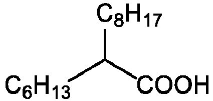 (the compound which has m=6 and n=8, obtained by oxidizing an alcohol, and an acid content is 97%).

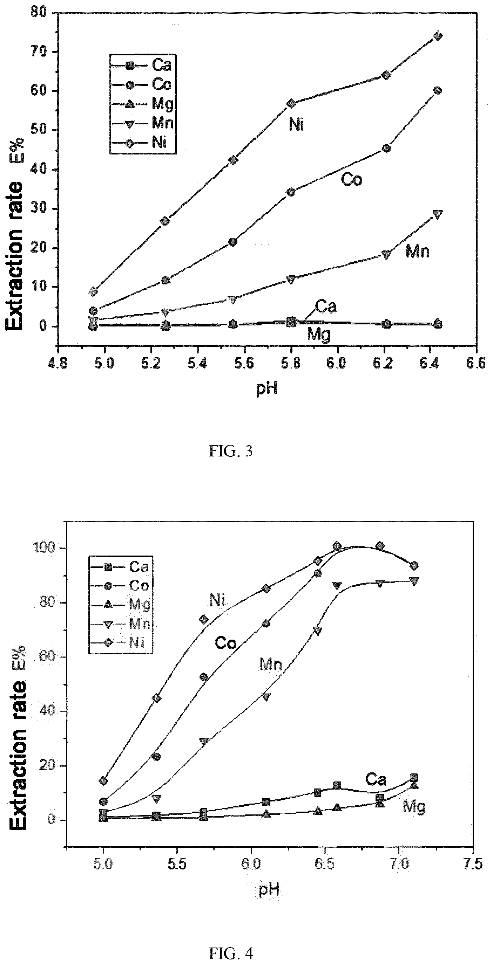

Extractant 196 was dissolved in Escaid 110 to prepared a 0.6 mol/L organic phase, and the battery material solution contained Ni (46.20 g/L), Co (20.56 g/L), Mn (23.93 g/L), Ca (0.43 g/L), and Mg (0.21 g/L). With a condition that the organic phase (O) and the aqueous phase (A) had a ratio of 5:1 and a ratio of 8:1, and controlling the pH by saponification using 10 mol/L NaOH, the extraction was carried out, and then the extraction rate E%-pH curves of Extractant 196 for each ion were obtained (FIG. 3 and FIG. 4).

It is shown in FIG. 3 and FIG. 4 that Ni, Co and Mn can be selectively extracted by the 196 from the battery material solution system at the pH of less than 7.2, and have high separation degree from Ca and Mg; when the O/A is 8:1 and the pH is greater than 6.8 (FIG. 4), Ni, Co and Mn can be almost completely extracted from the battery material solution system, while Ca and Mg have low extraction rate. It is demonstrated by this experiment that Extractant 196 has tangible application value in the recovery of nickel-cobalt-manganese ternary positive electrode material.

### Example 4

### Extraction performance of Extractant 196 for rare earth ions

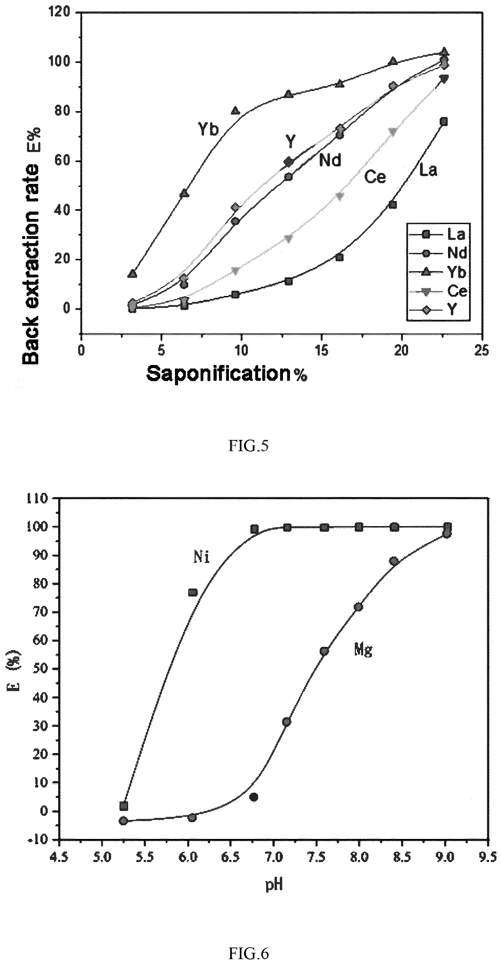

Extractant 196 was dissolved in dodecane to prepare a 2 mol/L organic phase, and a chloride solution of mixed ions including La³⁺, Ce³⁺, Nd³⁺, Y³⁺, and Yb³⁺ was prepared, and a concentration of each ion was 0.01 mol/L. With a condition that the organic phase and the aqueous phase had a ratio of 1:1, and controlling the pH by saponification using 10 mol/L ammonia, the extraction was carried out, and then the extraction rate E%-pH curves of Extractant 196 for each ion were obtained, as shown in FIG. 5.

It can be seen from FIG. 5 that the extraction ability of Extractant 196 for rare earth ions gradually decreases from heavy rare earths to light rare earths, the extraction sequence is consistent with that of P507, and the separation of rare earth ions can be achieved.

### Example 5

### Extraction with Extractant 191 for a magnesium-enriched nickel chloride solution

Extractant 191 was dissolved in dodecane to prepare a 0.31 mol/L organic phase, and an aqueous phase was the magnesium-enriched nickel chloride solution containing 1.33 g/L Ni and 4 g/L Mg. With a condition that the organic phase and the aqueous phase had a ratio of 1:1, and controlling the pH by saponification using 10 mol/L sodium hydroxide, the extraction was carried out, and then the extraction rate E%-pH curves of Extractant 191 for nickel and magnesium ions were obtained, as shown in FIG. 6.

It can be found from FIG. 6 that Extractant 191 extracts nickel in order before magnesium, and the separation coefficient between nickel and magnesium is about 833, thus indicating that the separation of nickel and magnesium can be achieved by Extractant 191.

### Example 6

### Extraction performance of Extractant 199 for rare earth ions

Extractant 199 is a hybrid extractant composed of four compounds as follows: 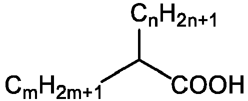 (m is 8, n is 2, and m+n=10), 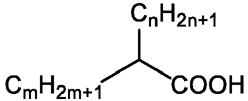 (m is 7, n is 9, and m+n=16), 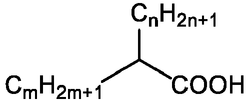 (m is 10, n is 8, and m+n=18), and 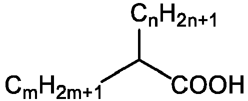 (m is 10, n is 10, and m+n=20).

In Extractant 199, the above four compounds have a volume ratio of 1:1:1:1, and an acid content is 92.6%.

Extractant 199 was dissolved in dodecane to prepare a 0.2 mol/L organic phase, and a nitrate solution of mixed ions including La³⁺, Ce³⁺, Nd³⁺, Y³⁺, and Yb³⁺ was prepared, and a concentration of each ion was 0.01 mol/L. With an O/A of 1:1 as well as saponification of 30% realized by 10 mol/L potassium hydroxide, the extraction rates for each ion were obtained and shown in Table 5.

**Table 5: Extraction rates of Extractant 199 for each ion**

| | Nd³⁺ | Ce³⁺ | Yb³⁺ | La³⁺ | Y³⁺ |
|---|---|---|---|---|---|
| pH=3.89 | 23.58 | 17.26 | 13.87 | 10.58 | 9.47 |

It can be seen from Table 5 that Extractant 199 first extracts rare earth ions except Y³⁺, which is expected to replace the naphthenic acid with unstable structure and properties.

### Example 7

### Saturation capacity test of Extractant 196 for Ni²⁺

Test method: 10 mL of a 196-dodecane organic phase with a concentration of 0.6 mol/L was added in a 50 mL separatory funnel, and saponified to 60% with 10 mol/L NaOH, and then 10 mL of a 50 g/L NiSO₄ aqueous phase was added, shaken and mixed for 15 min; the aqueous phase was separated, and then 10 mL of a fresh 50 g/L NiSO₄ aqueous phase was added, shaken and mixed for 15 min; the above operation was repeatedly carried out until the ion concentration in the aqueous phase did not change, and then the metal concentration of the organic phase was the saturation capacity of the extractant. The organic phase was back-extracted and the saturation capacity of Extractant 196 for Ni²⁺ was obtained to be about 16.9 g/L.

### Example 8

### Back extraction performance of Extractant 192 loaded with rare earth ions

Extractant 192 has a structure: 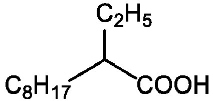 (m=8, n=2, obtained by oxidizing an alcohol, and an acid content is 95%).

Extractant 192 was dissolved in dodecane to prepare a 0.6 mol/L organic phase, and a 0.30 mol/L chloride solution of Lu³⁺ was prepared, and the organic phase was saponified to 60% with 9 mol/L ammonia and then subjected to extraction to obtain the 192 organic phase loaded with 0.10 mol/L Lu; with a condition that the organic phase and aqueous phase had a ratio of 1:1, the organic phase was back-extracted with 1.0 mol/L hydrochloric acid, and the back extraction rate was greater than 99%; the P507 organic phase loaded with Lu is generally back-extracted with 4 mol/L hydrochloric acid, and the primary back extraction rate is about 80%. It is demonstrated by the above results that the carboxylic acid compound shown in formula I can obtain high back extraction rates with lower back extraction acidity when applied to the extraction of rare earth metals.

### Solubility test of Extractant 194 and extractant Versatic 10 in the extraction system (Example 9 and Comparative Example 1)

### Example 9

Extractant 194 has a structure: 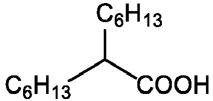 (m=6, n=6, obtained by oxidizing an alcohol, and an acid content is 99%).

Extraction: Extractant 194 and diluent Escaid 110 were prepared into a 0.62 mol/L solution, and an aqueous phase was 0.2 mol/L NiSO₄ solution; 100 mL of the organic phase was added in a 250 mL separatory funnel, 14 mol/L sodium hydroxide was added for saponification of 70%, 100 mL of the aqueous phase was added, and extraction equilibrium was carried out for 30 min.

Oil content test: the aqueous phase was separated out and added with H₂SO₄, and the [H⁺] concentration of the aqueous phase solution was about 1 mol/L. The CH₂Cl₂ was used for extraction (30 mL×3), and the CH₂Cl₂ layer was collected, dried with 1 g anhydrous Na₂SO₄ to remove the water in CH₂Cl₂, and filtered; the filtrate was subjected to rotary evaporation, and then the residue was dried with an oil pump for 30 min. The oil content which CH₂Cl₂ extracted out in the system was obtained by weighing the flask before and after the rotary evaporation.

### Comparative Example 1

This comparative example differs from Example 9 in that Extractant 194 was replaced with extractant Versatic 10 (commercially available, with an acid content of 98%).

The test results for Example 9 and Comparative Example 1 are shown in Table 6.

**Table 6: Solubility of Extractant 194 and Versatic 10 in the extraction system**

| | Versatic 10 | Extractant 194 | Blank Diluent |
|---|---|---|---|
| Equilibrium pH | 8.09 | 8.92 | - |
| Amount of Dissolved Organic Compound (mgAL) | 6000 | 115 | 46 |

Through the above tests, it can be seen that the oil content extracted after the blank diluent (with no extractant added, and other operations were the same as Example 8) reached equilibrium with the water phase is 46 mg/L, the oil content extracted after Extractant 194 reached extraction equilibrium at pH 8-9 is about 100 mg/L, and the oil content extracted after Versatic 10 reached extraction equilibrium at pH 8 is about 6000 mg/L. Versatic 10 has a large dissolution loss in the extraction system, which is likely to cause unstable process operation. When the carboxylic acid compound shown in formula I is used for metal ion extraction and separation, the problem is solved that extractant has a large solubility in the aqueous phase, the process operation is stable and the operation cost can be reduced by about 60 times.

### Solubility tests with Extractant 195 and extractant Versatic 911 in the extraction system (Example 10 and Comparative Example 2)

### Example 10

Extractant 195 and diluent Escaid 110 were prepared into a 0.62 mol/L solution, and an aqueous phase was a magnesium-enriched nickel chloride solution containing 1.33 g/L Ni and 4 g/L Mg; 100 mL of the organic phase was added in a 250 mL separatory funnel, 10 mol/L sodium hydroxide was added for saponification of 24%, 100 mL of the aqueous phase was added, and extraction equilibrium was carried out for 30 min.

Oil content test: the aqueous phase was separated out and added with H₂SO₄, and the [H⁺] concentration of the aqueous phase solution was about 1 mol/L. The CH₂Cl₂ was used for extraction (30 mL×3), and the CH₂Cl₂ layer was collected, dried with 1 g anhydrous Na₂SO₄ to remove the water in CH₂Cl₂, and filtered; the filtrate was subjected to rotary evaporation, and then the residue was dried with an oil pump for 30 min. The oil content which CH₂Cl₂ extracted out in the system was obtained by weighing the flask before and after the rotary evaporation.

### Comparative Example 2

This comparative example differs from Example 10 in that Extractant 195 was replaced with extractant Versatic 911 (commercially available, with an acid content of 98%).

The test results for Example 10 and Comparative Example 2 are shown in Table 7.

**Table 7: Solubility of Extractant 195 and Versatic 911 in the extraction system**

| | Versatic 911 | Extractant 195 | Blank Diluent |
|---|---|---|---|
| Equilibrium pH | 7.24 | 7.3 | - |
| Amount of Dissolved Organic Compound (mgAL) | 4680 | 75 | 46 |

Through the above tests, it can be seen that the oil content extracted after the blank diluent (with no extractant added, and other operations were the same as Example 10) reached equilibrium with the water phase is 46 mg/L, the oil content extracted after Extractant 195 reached extraction equilibrium at about pH 7.3 is about 75 mg/L, and the oil content for Versatic 911 is about 4680 mg/L. Versatic 911 has a large dissolution loss in the extraction system. When the carboxylic acid compound shown in formula I is used for metal ion extraction and separation, the problem that extractant has a large solubility in the aqueous phase is solved, the process operation is stable and the operation cost can be reduced.

### Comparative Example 3

This comparative example differs from Example 1 in that Extractant 191 was replaced with extractant Versatic 10 (commercially available, with an acid content of 98%).

The test results for Example 1 and Comparative Example 3 are shown in Table 8.

**Table 8: Separation coefficients of Extractant 191 and Versatic 10 for each ion**

| System | Metal ions | | | | | |
|---|---|---|---|---|---|---|
| | pH | βNi/Co | βNi/Mn | βNi/Ca | βNi/Mg | βNi/Zn |
| Extractant 191 | pH_{1/2} | 2.00 | 2.09 | 8.71 | 30.20 | 75.86 |
| Versatic 10 | pH_{1/2} | 1.56 | 1.61 | 6.89 | 23.00 | 57.89 |

It can be seen from Table 8 that the separation coefficients of Extractant 191 for each ion are higher by about 20-30% compared with Versatic 10 under the same test condition. With the half extraction pH condition, the separation coefficients of Extractant 191 were 30.2 and 75.86 for Ni/Mg and Ni/Zn, respectively, while the separation coefficients of Versatic 10 were 23.00 and 57.89 for Ni/Mg and Ni/Zn, respectively, which indicates that Extractant 191 has better ion separation effect compared with Versatic 10.

### Comparative Example 4

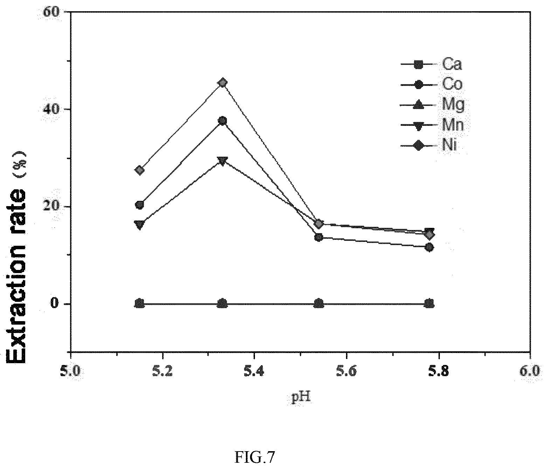

This comparative example differs from Example 3 in that Extractant 196 was replaced with a branched stearic acid which had eighteen carbon atoms (CORDA (UK), isostearic acid 3501, Prisorine 3501). The extractant was dissolved in Escaid 110 and prepared to a 0.6 mol/L organic phase, and a battery material solution contained Ni (46.20 g/L), Co (20.56 g/L), Mn (23.93 g/L), Ca (0.43 g/L), and Mg (0.21 g/L). With a condition that the organic phase (O) and the aqueous phase (A) had a ratio of 8:1, and controlling the pH by saponification using 10 mol/L NaOH, the extraction was carried out, and then the extraction rate E%-pH curves of the stearic acid for each ion were obtained (FIG. 7). By observing the experimental phenomenon where the extractant exists in the battery material solution system, it can be seen that when the aqueous phase equilibrium pH is greater than 5.33, the phase separation phenomenon becomes worse, the aqueous phase is turbid and viscous, and the organic phase gradually becomes colorless; it can be seen from FIG. 7 that when the aqueous phase equilibrium pH is greater than 5.33, the extraction capability of the extractant for metal ions decreases. The above results indicate that the extractant stearic acid has poor feasibility in the recovery of nickel-cobalt-manganese ternary positive electrode material.

### Comparative Example 5

This comparative example differs from Example 3 in that Extractant 196 was replaced with linear palmitic acid which had sixteen carbon atoms, and all other steps and parameters were the same. The results show that palmitic acid has poor solubility in Escaid 110 and the extraction experiments cannot be carried out.

The applicant has stated that although the detailed methods of the present application are illustrated by the embodiments in the present application, the present application is not limited to the detailed methods, which means that the present application is not necessarily rely on the detailed methods for implementation.

## Claims

1. An application of a carboxylic acid compound as an extractant, wherein the carboxylic acid compound has a structure shown in formula I: 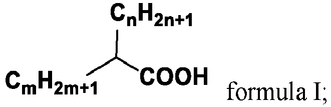 in formula I, 10≤m + n≤22, and both m and n are positive integers.

2. The application according to claim 1, wherein 10≤m + n≤20.

3. The application according to claim 1 or 2, wherein the m and the n are each independently an integer selected from 2 to 20.

4. The application according to any one of claims 1 to 3, wherein the m and the n are each independently an integer selected from 2 to 10.

5. The application according to any one of claims 1 to 3, wherein the carboxylic acid compound comprises any one or a combination of at least two of the following compounds:

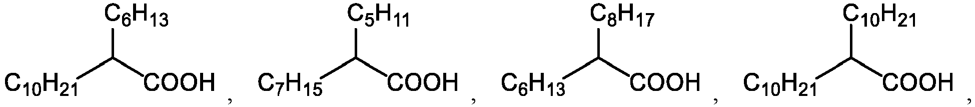

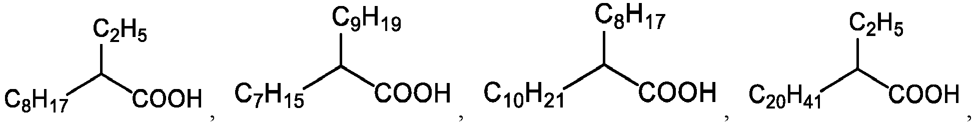

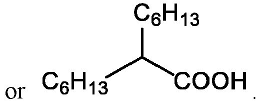

6. The application according to any one of claims 1 to 5, wherein the carboxylic acid compound is applied to extraction and separation of a metal ion;
optionally, the metal ion comprises any one or a combination of at least two of Fe³⁺, Al³⁺, Cu²⁺, Zn²⁺, Cd²⁺, Ni²⁺, Co²⁺, Mn²⁺, Ca²⁺, Mg²⁺, Li⁺, Na⁺, K⁺, Cr³⁺, Ga³⁺, In³⁺, Ti⁴⁺, Sc³⁺, Y³⁺, La³⁺, Ce³⁺, Pr³⁺, Nd³⁺, Sm³⁺, Eu³⁺, Gd³⁺, Tb³⁺, Dy³⁺, Ho³⁺, Er³⁺, Tm³⁺, Yb³⁺, or Lu³⁺.

7. The application according to claim 6, wherein the metal ion comprises a non-ferrous metal ion and/or a rare earth metal ion.

8. The application according to claim 6 or 7, wherein in the extraction and separation, an anion to match the metal ion in a feed solution to be extracted comprises any one or a combination of at least two of Cl⁻, SO₄²⁻ or NO₃⁻;
optionally, the carboxylic acid compound is applied to the extraction and separation of the metal ion from a used lithium-ion battery positive electrode material, a nickel-cobalt-containing waste residue or nickel laterite ore.

9. An extracting organic phase, wherein the extracting organic phase comprises the carboxylic acid compound shown in formula I: 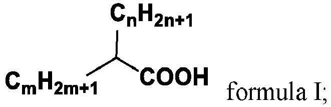 in formula I, 10≤m + n≤22, and both m and n are positive integers;
optionally, the extracting organic phase further comprises a diluent;
optionally, in the extracting organic phase, the carboxylic acid compound shown in formula I has a concentration of 0.1-2.0 mol/L.

10. A metal ion extraction method, comprising the following steps:
extracting a metal ion containing phase to be extracted by using the extracting organic phase according to claim 9, and back-extracting an obtained loaded organic phase to obtain a metal ion enriched solution and a regenerated organic phase;
optionally, a back extractant for the back-extracting comprises hydrochloric acid and/or sulfuric acid;
optionally, the hydrochloric acid used for the back-extracting has a concentration of 1-4 mol/L;
optionally, the sulfuric acid used for the back-extracting has a concentration of 0.5-4 mol/L;
optionally, the extracting organic phase and the phase to be extracted has a volume ratio of 1: 10-10: 1;
optionally, the loaded organic phase and the back extractant has a volume ratio of 1: 10-10: 1.
